# EUROPÄISCHE PATENTSCHRIFT

(11) **EP 2 739 592 B1**
(45) Veröffentlichungstag und Bekanntmachung des Hinweises auf die Patenterteilung: **29.03.2017**
(21) Anmeldenummer: 12753406.3
(22) Anmeldetag: 02.08.2012
(51) Int. Cl.: C07C 2/84, C07C 11/04, B01J 8/24, B01J 8/18, B01J 8/00

(54) **VERFAHREN ZUR OXIDATIVEN UMWANDLUNG VON GASFÖRMIGEN ALKANEN IN EINEM WIRBELSCHICHT-MEMBRAN-REAKTOR UND EIN REAKTOR ZUR DURCHFÜHRUNG DIESES VERFAHRENS**
METHOD FOR OXIDATIVELY CONVERTING GASEOUS ALKANES IN A FLUIDIZED BED MEMBRANE REACTOR AND A REACTOR FOR PERFORMING SAID METHOD
PROCÉDÉ DE TRANSFORMATION PAR OXYDATION D'ALCANES GAZEUX DANS UN RÉACTEUR À MEMBRANE À LIT FLUIDISÉ ET UN RÉACTEUR POUR METTRE EN OEUVRE CE PROCÉDÉ

(30) Priorität: 02.08.2011 DE 102011080294
(43) Veröffentlichungstag der Anmeldung: 11.06.2014
(73) Patentinhaber: Technische Universität Berlin, 10623 Berlin (DE)
(72) Erfinder: JASO, Stanislav, 60594 Frankfurt am Main (DE); ARELLANO-GARCIA, Harvey, 12157 Berlin (DE); GODINI, Hamid, 10555 Berlin (DE); WOZNY, Günter, 16548 Glienicke (DE)
(74) Vertreter: Maikowski & Ninnemann Patentanwälte Partnerschaft mbB
(86) Internationale Anmeldenummer: PCT/EP2012/065161
(87) Internationale Veröffentlichungsnummer: WO 2013/017664

(56) Entgegenhaltungen:
- WO-A2-2009/071463
- US-A1- 2005 036 940
- DESHMUKH ET AL: "Membrane assisted fluidized bed reactors: Potentials and hurdles", CHEMICAL ENGINEERING SCIENCE, OXFORD, GB, Bd. 62, Nr. 1-2, 18. November 2006 (2006-11-18), Seiten 416-436, XP005773247, ISSN: 0009-2509, DOI: 10.1016/J.CES.2006.08.062

## Beschreibung

Die vorliegende Erfindung betrifft ein Verfahren nach dem Oberbegriff des Anspruchs 1, einen Reaktor zur Durchführung dieses Verfahrens gemäß Anspruch 13.

Die oxidative Umwandlung von Alkanen umfasst die oxidative Kupplung von Methan als auch die oxidative Dehydrogenierung von höheren Alkanen.

Die oxidative Kupplung von Alkanen, insbesondere Methan, in dem so genannten OCM-Verfahren (Oxidative Methan Coupling) ist bekannt und wurde umfangreich untersucht. Grundlage dieses Verfahrens ist die Oxidation von Methan gemäß der Gleichung 2CH₄ + O₂ → C₂H₄ + 2 H₂O.

Diese Reaktion ist exothermisch und tritt üblicherweise bei hohen Temperaturen im Bereich zwischen 700°C bis 950°C auf. Im Verlaufe der Reaktion wird das Alkan heterogen an einer Katalysatoroberfläche unter Ausbildung freier Alkyl-Radikale aktiviert, welche anschließend in der Gasphase miteinander unter Ausbildung höherer, längerkettiger Kohlenwasserstoffe z.B. Ethan kuppeln. Die Ausbeute dieser Reaktion ist im Allgemeinen durch nicht-selektive Reaktionen der Alkylradikale mit der Oberfläche und dem Sauerstoff in der Gasphase reduziert.

Aufgrund der großen Mengen an natürlichen Gasvorkommen und den immer steigenden Preisen von Öl und seinen Derivaten, wie Naphta und Ethylen, besteht seit längerem der Wunsch, ein direktes Verfahren zur Herstellung von höheren Olefinen aus natürlich vorkommenden Gasen zu entwickeln und zu implementieren. Mit dem OCM-Verfahren erfolgt die katalytische und homogene Umwandlung von Methan in wertvolle Produkte, meistens Olefine wie Ethan und Ethen. Dieser Reaktionsprozess kann durch viele verschiedene Verfahren und Methoden umgesetzt werden. Es existieren ebenfalls Abwandlungen dieses Verfahrens, die den genannten OCM-Prozess als Teil eines allgemeinen Verfahrens zur Herstellung von Gasen beinhalten.

Aus vorhergehenden Untersuchungen des OCM-Verfahrens ist bekannt, dass eine hohe Selektivität bei der Umsetzung von Methan erreicht werden kann, wobei diese hohe Selektivität mit einer geringen Ausbeute an höheren Alkanen verbunden ist. Um eine hohe Selektivität zu bewirken, wird ein Gasstrom mit einem Überschuss an Methan im Vergleich zu Sauerstoff verwendet, um die Sauerstoffverfügbarkeit zu reduzieren, wodurch jedoch die Methanumsetzung bzw. Methankupplung reduziert wird. Dieser Ansatz ermöglicht eine Methanumsetzung in einem bestimmten, jedoch geringen Umfang, während der begrenzte Zustrom von Sauerstoff nicht ausreicht, um die während der Reaktion gebildeten Reaktionsprodukte unter Ausbildung von CO₂ zu verbrennen. Verschiedene theoretische und praktische Untersuchungen wurden durchgeführt, um zu ermitteln, ob die Verwendung von mehreren Einströmpunkten oder eine Gasverteilung mittels einer Membran zur Ausbeuteerhöhung der gewünschten Produkte möglich ist.

Gemäß der Kinetik des OCM-Verfahrens kann das Verhältnis zwischen der unerwünschten CO₂ Produktion und den gewünschten Reaktionen, z.B. Ethen- und Ethanproduktion, über die Verfügbarkeit des Sauerstoffs bzw. der Zugänglichkeit der gasförmigen Kohlenwasserstoffe, wie dem Methan, zum Sauerstoff beeinflusst werden. Zusätzlich ist eine Kontrolle der Selektivität mittels einer geeigneten Einstellung des Sauerstoffkonzentrationsprofils in der gesamten Reaktionszone bzw. Reaktionsbereich möglich.

Das im Folgenden beschriebene Verfahren umfasst die oxidative Umwandlung oder Konversion von gasförmigen Kohlenwasserstoffen, insbesondere Alkanen, wie z.B. die oxidative Kupplung von Methan unter Verwendung eines katalytischen Reaktors. Verschiedene Ansätze wurden auf diesem Gebiet in den letzten zwei Jahrzehnten verfolgt. So wurde unter anderem die Anwendung eines Wirbelschichtreaktors beschrieben. Ebenfalls wurde die Anwendung von Membranreaktoren detailliert untersucht.

Aus der WO 2009/071463 A2 ist ein Membranreaktor zur oxidativen Methankopplung entnehmbar, in welchem Sauerstoff durch eine gasdichte, gemischt leitende Membran der Reaktionszone zugeführt wird. Der methanhaltige Gasstrom wird in die Reaktionszone eingeleitet. Der für die Reaktion notwendige Katalysator ist auf Trägern wie z.B. Scheiben oder Rohren aufgebracht, die wiederum in der Reaktionszone angeordnet sind.

Darüber hinaus gibt es Berichte über die Anwendung von Wirbelschicht-Membran-Reaktoren zur Herstellung von Wasserstoff (US 7,141,231) durch Methanumwandlung. In diesen Anwendungen werden die Membrane zur Entfernung von Wasserstoff verwendet, jedoch nicht zur Verteilung eines Reaktanten.

Zur oxidativen Umsetzung von Kohlenwasserstoffen können auch Wirbelschicht-Membran-Reaktoren verwendet werden. In Desmukh et al. (Chem. Eng. Science, 2007, 62:416-436) wird u.a. ein Wirbelschicht-Membran-Reaktor zur partiellen Oxidation von Sauerstoff dargestellt.). In der Anwendung von Alonso et al. ( Catalysis Today, 2005, 104, 177) wird der kohlenwasserstoffhaltige Gasstrom in die Membran eingeführt, welche den Katalysator enthält, und Sauerstoff in das Wirbelschichtbett eingeführt. Zudem wird das Wirbelschichtbett durch den Sauerstoffstrom ausgebildet. Somit erfolgt in diesem Fall die eigentliche Oxidation von Butan in dem Festbett des Katalysators, der in den Membranen verpackt ist.

Der vorliegenden Erfindung liegt somit die Aufgabe zugrunde, ein Verfahren zur oxidativen Umwandlung von gasförmigen Alkanen, insbesondere zur oxidativen Kupplung von Methan, bereitzustellen, welches eine Erhöhung der Effizienz des selektiven Oxidationsprozesses ermöglicht.

Diese Aufgabe wird erfindungsgemäß durch ein Verfahren mit den Merkmalen des Anspruchs 1 gelöst.

Demnach wird ein Verfahren zur oxidativen Umwandlung von mindestens einem gasförmigen Alkan, bevorzugt von einem C1 bis C4 Alkan, insbesondere bevorzugt von Methan, in mindestens einem Wirbelschicht-Membran-Reaktor bereitgestellt. Der Wirbelschicht-Membran-Reaktor umfasst dabei mindestens eine erste Eintrittsleitung, mehr als eine zweite Eintrittsleitung, welche jeweils zumindest abschnittsweise innerhalb des Reaktors in Form einer Membran mit mindestens einer aktiven Membranfläche parallel zueinander angeordnet vorliegen, und mindestens ein Katalysatorbett.

Erfindungsgemäß wird mindestens ein erster Strom enthaltend das mindestens eine gasförmige Alkan durch die mindestens eine erste Eintrittsleitung in das mindestens eine Katalysatorbett in den Reaktor eingeleitet, wobei das Einleiten des ersten alkanhaltigen Stroms in das mindestens eine Katalysatorbett zur Ausbildung von mindestens einer Katalysator-Wirbelschicht in dem Wirbelschicht-Membran-Reaktor führt. Der erste alkanhaltige Strom stellt somit das fluidisierende Gas für den Katalysator dar.

Gleichzeitig wird erfindungsgemäß mindestens ein zweiter Strom enthaltend Sauerstoff oder ein sauerstoffhaltiges Gas in den Reaktor durch bzw. über mehr als eine zweite Eintrittsleitung eingeleitet, wobei der zweite Strom über die jeweilige mindestens eine aktive Membranfläche der zweiten Eintrittsleitung in die mindestens eine Katalysator-Wirbelschicht des Reaktors eingeführt bzw. eingeleitet wird.

Die Umsetzung von Alkanen wie z.B. Methan wird vorliegend somit bevorzugt in einer Wirbelschicht aus Katalysatormaterial durchgeführt und nicht in einem Festbett.

Das vorliegende Verfahren wird somit in einem Wirbelschicht-Membran-Reaktor mit einer feinteiligen Verteilung der Reaktanten durchgeführt, wodurch eine höhere Ausbeute der gewünschten Produkte und gleichzeitig eine isotherme Bedienung ermöglicht werden. Insbesondere wird in dem vorliegenden Verfahren ein Wirbelschicht-Membran-Reaktor verwendet, wobei die Eigenschaft von gasdurchlässigen Membranen zur feinen Verteilung von Sauerstoff in dem Wirbelschichtbett des Katalysators ausgenutzt wird.

Die vorliegende Erfindung implementiert somit eine Reaktorstruktur, die die bekannten Konzepte eines Wirbelschicht- Reaktors und eines Membran-Reaktors verbindet, um die Effizienz und Umsetzung der oxidativen Umwandlung von Alkanen, insbesondere Methan im Rahmen des so genannten OCM Prozesses zu erhöhen. Die Verwendung eines Wirbelschicht-Membran-Reaktors ermöglicht somit aufgrund der Verwendung eines gasdurchlässigen Materials als Membran und aktiver Membranfläche eine feine Verteilung von Sauerstoff oder eines sauerstoffhaltigen Gases innerhalb des Wirbelschichtbettes, um eine selektive Prozessführung zu gewährleisten. Gleichzeitig ermöglicht die aus dem Katalysatorbett bestehende Wirbelschicht die Bereitstellung einer homogenen katalytischen Zone und eines isothermen Katalysatorbettes, was wesentlich für die exotherme oxidative Umwandlung ist. Beide der genannten Maßnahmen verbessern die Verfahrenseffizienz bei der Produktion von wertvollen höheren Produkten, wie zum Beispiel Ethan und Ethen durch die oxidative Kupplungsreaktion. Insgesamt gewährleistet das vorliegende Verfahren einen isothermen Verlauf bei Einleiten von unverdünnten Gasen und eine Erhöhung der Ausbeute an Ethan und Ethen im Vergleich zu klassischen Wirbelschichtreaktoren.

Es sei angemerkt, dass eine Kombination von Membranreaktor und Wirbelschichtbettreaktor, wie vorliegend verwirklicht, eine Vielzahl von Maßnahmen und Anpassungen erfordert, um den gewünschten Effizienzanstieg zu gewährleisten. So gibt es eine Vielzahl von verschiedenen Möglichkeiten zur Kombination der beiden Konzepte, wobei jedoch nur die vorliegend beschriebenen Kombinationsansätze garantiert eine Verbesserung der Reaktionseffizienz garantieren.

Im Rahmen der vorliegenden Erfindung wird unter dem Begriff der "aktiven Membranfläche" die Membranfläche verstanden, die unmittelbar mit der Wirbelschicht des Reaktorbettes in Kontakt steht und in dieses eintaucht. Somit stellt die aktive Membranfläche den Abschnitt der Membran dar, über die der Sauerstoff bzw. das sauerstoffhaltige Gas unmittelbar in das Katalysatorbett eingeführt bzw. eingeleitet wird.

In dem vorliegenden Verfahren werden die Gasströme enthaltend ein gasförmiges Alkan, wie zum Beispiel Methan, und Sauerstoff bzw. ein sauerstoffhaltiges Gas separat, d.h. räumlich getrennt voneinander, in den Reaktor eingeführt.

So erfolgt der Eintrag des alkanhaltigen Gasstroms bevorzugterweise über den Boden des Reaktors, das heißt an der Unterseite des Reaktors in denselbigen. Das Einleiten dieses ersten alkanhaltigen Stroms in das mindestens ein Katalysatorbett führt wie oben ausgeführt zur Ausbildung von mindesten einer Wirbelschicht in dem vorliegenden Wirbelschicht-Membran-Reaktor. Der alkanhaltige Strom wie z.B. ein methanhaltiger Strom kann auch Spuren von Sauerstoff enthalten, wobei bevorzugt jedoch sauerstofffreies Alkan verwendet wird.

Der zweite Gasstrom enthaltend Sauerstoff oder ein sauerstoffhaltiges Gas wird über die zweite Eintrittsleitung und daran ausgebildeten aktiven Membranfläche der Membran bevorzugt von der Oberseite des Reaktors, d.h. bevorzugt mit gegenläufiger Strömungsführung in die Wirbelschicht eingeleitet und reagiert in der Wirbelschicht mit dem Alkan, insbesondere Methan, in einem Bereich mit einer gleichförmigen Temperaturverteilung durch Kontaktierung mit den geeigneten Katalysatoren, die für die oxidativen Reaktionen verwendet werden. Das sauerstoffhaltige Gas kann z.B. aus einer Mischung von Sauerstoff und Alkan wie Methan bestehen.

Die Einleitung des Sauerstoffs über die Membran an der zweiten Eintrittsleitung wird bevorzugt entgegengesetzt zur Flussrichtung des alkanhaltigen Stroms, d.h. im Gegenstrom, in die Wirbelschicht eingetragen. Es ist aber auch denkbar, Sauerstoff im Gleichstrom oder als Dead-end Strom (d.h. kein durchgängiger Strom) oder auch im Kreuzstrom zur Durchflussrichtung des alkanhaltigen Gases einzuführen. Entsprechende Ausführungsformen sind weiter unten beschrieben.

Die Zusammensetzung der in den Reaktor einzuführenden Gasströme ist insbesondere abhängig von der Reaktorgröße. So kann es vorteilhaft sein 30% des für die Oxidation notwendigen Sauerstoffs von unten, d.h. durch die Unterseite des Reaktors, und 70% des Sauerstoffs über die Membran in den Reaktor einzuleiten. In diesem Fall ist es möglich, die Reaktorgröße zu minimieren ohne negative Auswirkungen auf die Selektivität oder Ausbeute der Umsetzung befürchten zu müssen.

Nach Umsetzung der Reaktanten mit einer optimalen Kontaktzeit strömen bzw. verlassen die produzierten höheren Alkane, Alkene oder weiteren ungesättigten Kohlenwasserstoffverbindungen mit Resten von Reaktanten, aber bevorzugterweise ohne Sauerstoff, den Reaktionsbereich und werden sofort gekühlt, um weitere ungewünschte Seitenreaktionen, wie Reforming der Alkane, zu vermeiden.

Das vorliegende Verfahren ermöglicht eine hohe Selektivität, Ausbeute und Umsetzung unter gleichzeitiger Berücksichtigung von praktischen Betrachtungen, wie einer Hot-Spot-Bildung und Katalysator-Rezirkulation und/oder -Reaktivierung. Das vorliegende Verfahren kann alleine oder in Kombination mit anderen Reaktorkonzepten, Trennungskonzepten mit oder ohne Recyclingströmen verwendet werden

Bevorzugterweise ist lediglich ein begrenzter Bereich der Membran permeabel bzw. gasdurchlässig. Der gasdurchlässige Bereich der Membran ist bevorzugt komplett in das Wirbelbett eingetaucht; dieser gasdurchlässige Bereich ist somit die aktive Membranfläche. Bevorzugterweise sollte vermieden werden, dass der gasdurchlässige Membranbereich sich außerhalb des Wirbelbetts befindet, da ansonsten der austretende Sauerstoff in der Wirbelbettfreien bzw. Post-Reaktionszone eine weitere, hier jedoch ungewünschte Oxidation der Produkte oder sogar eine Explosion verursachen würde.

Der nicht-gasdurchlässige Bereich der Membran innerhalb des Reaktors ist bevorzugterweise außerhalb des Reaktorbetts. Dabei muss es sich nicht zwingend um ein nicht-gasdurchlässiges Membranmaterial, welches spezifisch modifiziert wurde, handeln, sondern dieser nicht-gasdurchlässige Bereich kann auch aus einem anderen Material, z.B. Metall oder Metalllegierung bestehen und kann ein Abschnitt der zweiten Eintrittsleitung sein.

Der Bereich bzw. die Fläche der gasdurchlässigen Membran, die in das Wirbelbett eintaucht ist in erster Linie von der Reaktorgröße bzw. den Reaktordimensionen abhängig. So ist es wünschenswert, die in das Wirbelbett eingetauchte Membranfläche zu maximieren, um einen optimalen Sauerstoffeintrag zu haben.

Eine Möglichkeit zur Maximierung der eingetauchten Membranfläche besteht darin, mehr als eine Membran z.B. bis zu 10 000 abhängig von der Reaktorgröße zu verwenden. Diese können parallel zueinander im Reaktor angeordnet sein. Bei dieser Ausführungsform ist jedoch zu beachten, dass der Abstand zwischen den Membranen nicht zu klein ist, d.h. dass die Packungsdichte der Membranen nicht zu groß ist. Eine zu hohe Packungsdichte kann zu einer Beeinflussung der Hydrodynamik des Wirbelbetts führen, da nicht genug Raum bzw. Platz für die Verwirbelung des Reaktorbetts verbleibt und eine Kanalbildung in Membrannähe auftreten kann. Aus diesem Grunde sollte der Abstand der Membranen voneinander mindestens 1 cm betragen.

Die zum Einsatz kommenden Membranen können einen Durchmesser bis zu 30 mm, bevorzugt 5 mm bis 25 mm, insbesondere bevorzugt 10 mm bis 20 mm aufweisen. Generell sind die Dimensionen der Membranen aber abhängig von der Größe des verwendeten Reaktors und werden entsprechend angepasst. Dies liegt im Ermessen des Fachmanns.

Der Sauerstoffeintrag in das Wirbelbett über die aktive Membranfläche kann aber auch wie folgt beeinflusst werden.

In einer Ausführungsform des vorliegenden Verfahrens kontaktiert die mindestens eine aktive Membranfläche, d.h. gasdurchlässige Membran, vollständig das Katalysatorbett, d.h. die aktive Membranfläche ist in Berührung mit dem Katalysatorbett bzw. taucht in dieses ein. Dabei ist es möglich, die Menge bzw. die Fläche der mindestens einen das Katalysatorbett kontaktierenden aktiven Membranfläche zur Einstellung des Flusses des mindestens zweiten Sauerstoff enthaltenden Stroms anzupassen oder zu variieren, so dass die Menge bzw. das Volumen des in das Katalysatorbett über die aktive Membranfläche einströmenden sauerstoffhaltigen Gases und damit die Sauerstoffkonzentration im Katalysatorbett gezielt einstellbar ist. Diese Anpassung bzw. Einstellung des Sauerstoffstroms in das Katalysatorbett kann in einer Ausführungsform über eine Hülle bzw. einen Mantel erfolgen, der die Membran und somit einen Abschnitt der zweiten Eintrittsleitung umgibt und entlang dieser bewegbar ist. Mit anderen Worten, eine derartige Hülle ist beweglich mit der zweiten Eintrittsleitung verbunden und kann in beliebiger Weise entlang der Eintrittsleitung bzw. entlang des die Membran bildenden Abschnitts der Eintrittsleitung verschoben werden und so jeweils einen bestimmten Teil der aktiven Membranfläche zum Sauerstoffeintrag in das Katalysatorbett freigeben. Eine gezielte Beeinflussung des Sauerstoffstroms in das Katalysatorbett ist somit möglich.

Eine Regulierung des Sauerstoffstroms ist ebenfalls über die Porengröße der verwendeten Membran möglich. So sind Porengrößen von kleiner 500 nm, insbesondere zwischen 2 nm und 20 nm bevorzugt. Bei der Auswahl der geeigneten Porengröße ist zu beachten, dass ein Druckverlust an der Membran zu einem Rückfluss des Methans durch die Membran bewirken könnte. Dies kann durch die Verwendung von geeigneten Porengrößen vermieden werden.

In einer weiteren Ausführungsform ist es auch möglich, den Sauerstoffstrom in das Katalysatorbett über die aktive Membranfläche durch Hoch- und Runterfahren der mindestens zweiten Eintrittsleitung innerhalb des Reaktors einzustellen. Durch ein Hoch- und Runterfahren der zweiten Eintrittsleitung kann gezielt die Menge bzw. Fläche der Membran und somit der aktiven Membranfläche eingestellt werden, die mit dem Katalysatorbett in Kontakt ist bzw. in dieses eintaucht. Der Teil der zweiten Eintrittsleitung, der nicht in das Katalysatorbett eintaucht, ist in dem oberen, von Katalysator und Wirbelschicht freien Bereich des Reaktors angeordnet bzw. steht in einem beweglichen Kontakt mit der Reaktorumgebung.

In einer anderen Ausführungsform wird der Sauerstoffstrom durch die aktive Membran extern von außerhalb des Reaktors unter Verwendung von geeigneten Ventilen z.B. an der zweiten Eintrittsleitung geregelt.

In einer weiteren Ausführungsform ist es ebenfalls möglich, den Sauerstofffluss durch die Membranen durch die spezifische Anordnung der Membranen in dem Reaktor zu beeinflussen bzw. zu regulieren. Ist die Membran zentral innerhalb des Reaktors angeordnet, ist der Sauerstofffluss größer, da von allen Seiten zugängig, als wenn die Membran nahe zur Reaktorwand platziert wird. Im letzteren Fall ist der Sauerstoffeintrag geringer, um eine erhöhte lokale CO₂ Produktion zu verhindern.

In einer Ausführungsform bestehen das Katalysatorbett und damit die daraus gebildete Wirbelschicht aus Katalysatorpartikeln mit einer Größe zwischen 10 µm und 10 mm, bevorzugt zwischen 75 µm und 500 µm, insbesondere bevorzugt zwischen 100 µm and 300 µm.

Der Wirbelschicht-Membran-Reaktor wird bevorzugt in der bekannten Wirbelschichtweise betrieben, nämlich homogen, blasenförmig, mit einer schnellen Verwirbelung bzw. Fluidisierung, in einer Steigrohr-Fallrohr-Konfiguration oder durch pneumatische Förderung und/oder als klassischer Wirbelbett-Reaktor. Bevorzugt ist jedoch die Blasenbildung in der Wirbelschicht bzw. das Sprudeln der Wirbelschicht.

Die minimale Fluidisierungsgeschwindigkeit (U_{mf}) kann nach Wen und You kalkuliert werden, wobei der Bereich zwischen (0.5 - 300) x u_{mf}, bevorzugt (1-15) x u_{mf} für das verwendete Katalysatorbett liegen sollte. Für kleine Partikel mit einem mittleren Partikeldurchmesser bis zu ca. 75 µm beträgt die Fluidisierungsgeschwindigkeit typischerweise 0,1 cm/s bis 15 cm/s und für größere Partikel mit einem mittleren Partikeldurchmesser von bis zu 300 µm 5 cm/s bis 8 m/s.

Als geeigneter Katalysator kann jeder für die oxidative Umwandlung von Kohlenwasserstoffen bekannte Katalysator aus der Gruppe der X_{P}Y_{R}Z_{S}Q_{T}/M_{A}O_{B}-Katalysatoren verwendet werden. Geeignete Katalysatoren sind z. B. in der US 4,754,091 aufgelistet. X, Y, Z und Q sind dabei bevorzugterweise ausgewählt aus den folgenden Elementen oder deren Oxide, Hydroxide und/oder Carbonate, Legierungen oder andere Verbindungen enthaltend eines oder mehrere der folgenden Elemente: Li, Na, K, Rb, Cs, Fr, Be, Mg, Ca, Sr, Ba, Y, Bi, Rh, Ru, La, Pb, W, Mn, Al, Ag, Au, Pt, Ir, Nb, Pd, Zr, Sb, Te, Ga, Ni, Cu, Sn, Fe, Co, Mo, Te, Cr, und/oder V. Die Indizes P, R, S und T reflektieren deren molares Verhältnis in dem Katalysator in dem Bereich von 0,001 bis 1. Die Summe der molaren Komponenten der unterschiedlichen Elemente in dem Katalysator muss jeweils 1 betragen. Als geeignete Katalysatoren sei beispielhafterweise auf die folgenden Katalysatoren hingewiesen: La₂O₃/CaO, Na-Mn-W/SiO₂, La-Sr/CaO, Li/MgO, Na/CaO, EaO/Ga₂O₃, La/MgO, La₂O₃-CeO₂, Sm₂O₃, Sm/La₂O₃.

Die Katalysatoren können ohne Träger oder mit einem Träger hergestellt aus einem Oxid des Typs M_{A}O_{B} verwendet werden, wobei M ausgewählt ist aus einer der folgenden Komponenten Mg, Ca, Sr, La, Zr, Si, Al, Y, Sm, Ti oder einem anderen keramischen Oxid-Trägermaterial. Des Weiteren können unterschiedliche inerte Packungen wie z. B. Quarzglas, Silizium, Aluminium, Magnesium, Titan und Gasverdünnungen durch z. B. Sauerstoff, Argon, Helium oder andere inerte Gase verwendet werden.

Die Höhe der Wirbelschicht bzw. des verwirbelten Katalysators ist abhängig von der Größe des verwendeten Reaktors, des verwendeten Katalysators und/oder der eingestellten Flussrate. Von Bedeutung dabei ist, dass die Katalysatorpartikel bzw. das Katalysatorbett zumindest im oberen Bereich des Katalysatorbettes verwirbelt sein sollten. Die Einstellung der genannten Parameter sind einem Fachmann vertraut und routinemäßige Vorgänge.

In einer weiteren Ausführungsform kann die Wirbelschicht nicht nur durch den Alkanstrom, sondern auch durch weitere inerte Verdünnungsgase oder reaktive Fluidisierungsgase hergestellt werden.

Wie bereits oben erwähnt, ist mindestens ein Abschnitt der zweiten Eintrittsleitung zum Einleiten des sauerstoffhaltigen Gases in das Wirbelschichtbett in Form einer Membran ausgebildet. Diese Membran kann bevorzugter Weise aus einem porösem, perforierten oder anderweitig gasdurchlässigen Material bestehen, insbesondere aus einem keramischen, metallischen, porös-keramischen, porös-metallischen und/oder perforierten metallischen Material bestehen. Als Membran ist somit jedes gasdurchlässige Material geeignet, das den oxidativen Reaktionsbedingungen, hohen Temperaturen, hohen Drücken und Abrieb durch die Wirbelschicht widerstehen kann. Die Membran dient der Verteilung des sauerstoffhaltigen Gases und kann somit auch als ein Verteiler oder eine Verteilungsvorrichtung angesehen werden.

Auch ist es möglich, Membranen mit oder ohne Modifikationen wie Imprägnierung, partielle Blockierung, Kalzinierung etc. zu verwenden. Es ist ebenfalls vorstellbar, dass die Membranen mit oder ohne eine katalytische Schicht in dem vorliegenden Verfahren verwendet werden und als eine selektive Membran oder als ein Gasverteiler fungieren.

In einer bevorzugten Ausführungsform ist die Membran parallel oder vertikal in Bezug auf die Durchflussrichtung des alkanhaltigen ersten Gasstromes durch den Reaktor innerhalb des Reaktors angeordnet. Das heißt, die mindestens eine Membran ist im Reaktor parallel oder vertikal in Bezug auf die Reaktorhöhe bzw. -länge angeordnet.

Die parallele Anordnung der Membran in Bezug auf die Durchflussrichtung des ersten alkanhaltigen Gasstromes kann entweder in Form einer so genannten Dead-end-Konfiguration oder einer Durchflusskonfiguration erfolgen. Somit ist ein Einleiten des sauerstoffhaltigen Gases durch die Membran im Gleichstrom, Gegenstrom oder Kreuzstrom mit dem gasförmigen alkanhaltigen Gas, das zur Ausbildung der Wirbelschicht dient, möglich.

Auch ist es bevorzugt mehr als eine zweite Eintrittsleitung und damit mehr als eine Membran im vorliegenden Verfahren zu verwenden. Generell denkbar sind bis zu 10 000 parallel angeordnete Membranen. Die Anzahl der zum Einsatz kommenden Membranen wird in Abhängigkeit von der Reaktorgröße und -design ausgewählt.

Darüber hinaus ist es möglich, das vorliegende Verfahren lediglich für eine Reaktion, d.h. eine oxidative Kupplung, oder auch in Kombination mit weiteren chemischen Reaktionen wie z.B. einer oxidativen Dehydrogenierung, zu kombinieren. Die vorgeschlagene Kombination mit weiteren Reaktionen kann entweder in verschiedenen Reaktoren erfolgen, d.h. jede der einzelnen Reaktionen wird in einem separaten Reaktor ausgeführt, oder ähnliche Reaktionen können in einem gemeinsamen Reaktor durchgeführt werden, was zu erheblichen Einsparungen bezüglich des notwendigen Reaktormaterials führt.

Bevorzugterweise wird das erfindungsgemäße Verfahren bei Reaktionstemperaturen im Bereich zwischen 350°C und 1300°C, bevorzugt im Bereich zwischen 500°C und 1000°C, insbesondere bevorzugt zwischen 650°C und 900°C durchgeführt.

Der verfahrensgemäße Druck liegt in einem Bereich zwischen 0,2 bar und 100 bar, bevorzugt zwischen 1 barund 20 bar, insbesondere bevorzugt zwischen 1 bar und 6 bar.

Der erste alkanhaltige Gasstrom enthält bevorzugterweise 0,1 % bis 100 % Alkan, 5 % bis 100 % Alkan, insbesondere bevorzugt 20 % bis 80 % Alkan.

In einer Ausführungsform enthält der sauerstoffhaltige zweite Gasstrom 0,1 % bis 100 % Sauerstoff, bevorzugt 2 % bis 70 % Sauerstoff, insbesondere bevorzugt 5 % bis 20 % Sauerstoff.

Unterschiedliche Materialien können zur Herstellung des Wirbelschichtreaktors verwendet werden, typischerweise Keramik, Metall oder Glas. In einer bevorzugten Ausführungsform ist der Reaktor aus einem korrosions- und druckbeständigem Material, bevorzugterweise aus einer Eisen-Chrom-Legierung, Hastelloy X (Nickel-Chrom-Eisen-Molybdän-Legierung) oder einer ähnlichen temperaturstabilen Legierung hergestellt. Insbesondere sollte das Reaktormaterial kein Nickel enthalten, um eine Nickel-katalysierte Umlagerungsreaktion der Reaktanten und Produkte zu vermeiden. Der Reaktor kann ebenfalls mit Quartz oder anderen inerten keramischen Materialien zur Vermeidung von Reaktionen an der Reaktorwand ausgelegt sein.

Das vorliegende Verfahren wird demnach in einem Wirbelschicht-Membran-Reaktor durchgeführt, welcher
- mindestens ein Katalysatorbett,
- mindestens eine erste Eintrittsleitung zum Einleiten von mindestens einem ersten Strom enthaltend mindestens ein erstes Gas, insbesondere ein gasförmiges Alkan, in das mindestens eine in dem Reaktor angeordnete Katalysatorbett und
- mehr als eine zweite Eintrittsleitung, welche jeweils zumindest abschnittsweise innerhalb des Reaktors in Form einer Membran mit einer aktiven Membranfläche parallel zueinander angeordnet vorliegen, zum Einleiten von mindestens einem zweiten Strom enthaltend mindestens ein zweites Gas, insbesondere ein sauerstoffhaltiges Gas, über die aktive Membranfläche in das mindestens eine Katalysatorbett des Reaktors umfasst.

Dabei ist in einer Ausführungsform in dem vorliegenden Reaktor der als Membran ausgebildete Abschnitt der zweiten Eintrittsleitung bevorzugterweise parallel oder senkrecht zur Durchflussrichtung des ersten Gasstroms durch den Reaktor angeordnet.

Darüber hinaus ist der als Membran ausgebildete Abschnitt der zweiten Eintrittsleitung zumindest teilweise oder vollständig von dem Katalysatorbett bedeckt, bzw. taucht zumindest teilweise oder vollständig in das insbesondere verwirbelte Katalysatorbett ein. Wesentlich ist, dass die aktive Membranfläche, d.h. die gas-durchlässige Membranfläche komplett in das Katalysatorbett eintaucht.

Erfindungsgemäß sind der als Membran ausgebildete Abschnitt der zweiten Eintrittsleitung und damit auch die aktive Membranfläche mittels einer Steuereinrichtung innerhalb des Reaktors bewegbar. Somit kann der vorliegende Reaktor eine Steuereinrichtung zur Bewegung des als Membran ausgebildeten Abschnitts einer Eintrittsleitung für einen Gasstrom innerhalb eines Reaktors aufweisen.

In einer Ausführungsform des Reaktors sind das Wirbelbett und die Anordnung der aktiven Membranfläche innerhalb des Reaktors so gestaltet, dass vom unteren Reaktorende her gesehen das erste untere Drittel des Wirbelbettes keine Membran enthält. Die gasdurchlässige Membran befindet sich bevorzugt in dem mittleren Drittel des Wirbelbettes. In den verbliebenen oberen Drittel Wirbelbettes, d.h. im oberen Abschnitt des Wirbelbettes, ist die Membran hingegen bevorzugt nicht-gasdurchlässig.

Mit anderen Worten der aktive, gasdurchlässige Abschnitt der Membran befindet sich bevorzugt im mittleren Drittel der Wirbelschicht. Diese Angaben sind lediglich als Näherung anzusehen. So ist es ebenfalls möglich, dass ausgehend von einer Reaktorbetthöhe von 1 m von unten gesehen in den ersten 15 cm bis 40 cm ein freies Wirbelbett vorhanden ist, anschließend in einer Höhe von 40 cm bis 80 cm des Reaktorbettes der gasdurchlässige Membranabschnitt angeordnet ist und in den verbleibenden 20 cm der nicht-gasdurchlässige Membran- oder Leitungsabschnitt angeordnet ist.

Der untere Abschnitt bzw. Bereich des Wirbelbettes sollte demnach keine Membran enthalten. Dies ist durch die sog. "Channeling" Effekte bedingt, die in Membrannähe auftreten bzw. auftreten können, wodurch die Möglichkeit besteht, dass die Reaktanten ohne Umsetzung bzw. Reaktion den Reaktor passieren.

Im oberen Abschnitt des Wirbelbettes sollte demnach keine gasdurchlässige Membran oder Leitung angeordnet sein, so dass in diesem Bereich kein Sauerstoff bzw. sauerstoffhaltiges Gas in das Wirbelbett eintreten kann. Dies erlaubt eine komplette Sauerstoffumsetzung im Wirbelbett, so dass kein Sauerstoff in der Post- bzw. Nach-Reaktionszone des Reaktors enthalten ist, wodurch eine weitergehende Oxidation und die Gefahr von Explosionen vermieden wird.

In einer weiteren Ausführungsform kann der vorliegende Reaktor mit anderen Reaktortypen, insbesondere einem Festbettreaktor, simulierten Wanderbettreaktor oder anderer für die oxidative Kopplung geeigneten Reaktortypen mit oder ohne Recyclingstrom kombiniert werden. Somit kann der Wirbelschicht-Membran-Reaktor als Einzelreaktor verwendet werden, in welchen reine Gasströme eingeführt werden, oder als Teil eines Prozesses, in welchem der vorliegende Reaktor vor, nach oder zwischen weiteren Prozesseinheiten verwendet wird.

Die Erfindung wird nachfolgend unter Bezugnahme auf die Figuren der Zeichnungen an mehreren Ausführungsbeispielen näher erläutert. Es zeigen:
- Figur 1: eine schematische Ansicht eines Wirbelschicht-Membran-Reaktors gemäß einer ersten Ausführungsform,
- Figur 2a: eine schematische Ansicht einer ersten Ausführungsform der Membran in dem erfindungsgemäßen Reaktor,
- Figur 2b: eine schematische Ansicht einer zweiten Ausführungsform der Membran in dem erfindungsgemäßen Reaktor,
- Figur 2c: eine schematische Ansicht einer dritten Ausführungsform der Membran in dem erfindungsgemäßen Reaktor,
- Figur 2d: eine schematische Ansicht einer vierten Ausführungsform der Membran in dem erfindungsgemäßen Reaktor, und
- Figur 3: eine schematische Ansicht von Wirbelschicht-Membran-Reaktoren mit verschiedenen Ansätzen der Sauerstoffzufuhr.

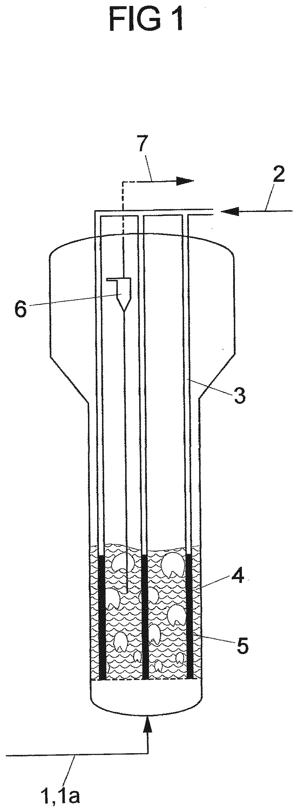

Ein allgemeiner Aufbau des vorliegenden Wirbelschicht-Membran-Reaktors ist in Figur 1 gezeigt.

Das Alkan z. B. Methan, wird am Boden des Reaktors bzw. der Unterseite des Reaktors in den selbigen über die Eintrittsleitung 1 eingeführt. Das Gas kann in dem Reaktor entweder durch eine poröse oder perforierte Platte, poröse oder perforierte Schläuche, Glockenboden, Düsen oder jede andere geeignete Vorrichtung zur Gasverteilung in den Reaktor eingeführt werden.

Der alkanhaltige Gasstrom 1 a enthält verdünntes oder reines Methan, welches durch das Katalysatorbett 4 durchgeführt wird. Das Katalysatorbett am Boden des Reaktors kann, muss jedoch nicht, verwirbelt sein.

Ein reiner oder verdünnter Sauerstoffgasstrom 2 wird über die zweite Eintrittsleitung 3 in die als Schläuche ausgebildete Membranen 5 eingeführt. Im vorliegenden Fall der Ausführungsform der Figur 1 wird der Sauerstoffgasstrom in insgesamt drei Leitungen nach Eintritt in den Reaktor aufgeteilt und erreicht somit drei zueinander parallel angeordnete Membranen 5. Der Sauerstoffeintrag erfolgt gemäß Figur 1 über Dead-end-Membranen, d.h. der gesamte in die Membranen 5 eingeführte Sauerstoffstrom wird in das Katalysatorbett 4 eingeleitet.

Die Eintrittsleitungen 3 mit den Abschnitten der Membranen 5 werden in das Wirbelschichtbett eingeführt (siehe Fig. 1), jedoch ist nur ein geringer Abschnitt der Membranen 5 gasdurchlässig bzw. steht als aktive Membranfläche zur Verfügung. Dieser gasdurchlässige Abschnitt bzw. die aktive Membranfläche befindet sich innerhalb des Reaktionsreaktors und ist in das aus den verwirbelten Katalysatorpartikeln gebildete Wirbelschichtbett eingetaucht.

Der Sauerstoffgasstrom 2 wird durch die durchlässige Membran 5 in das Katalysatorbett 4 bzw. die daraus gebildete Wirbelschichtbett eingeführt, in welchem es mit dem Methanstrom 1 a in Gegenwart des Katalysators, der das Wirbelschichtbett 4 bildet, unter Ausbildung von höheren Alkenen und Alkenen, z. b. Ethylen und Ethan, reagiert.

Die Katalysatorpartikel am Boden des Reaktors müssen jedoch nicht in einem Wirbelschichtzustand vorliegen. Auch ist es möglich, den Katalysator erst dann zu verwirbeln, nachdem der Sauerstoffstrom in das Katalysatorbett eingeführt würde.

Wesentlich ist, dass das Katalysatorbett zumindest in dem oberen Bereich des Katalysatorbettes, in welchem die Reaktion stattfindet, verwirbelt wird, um das Wirbelbett in einen isothermen Zustand zu halten und ein Ausreißen bzw. Streuen zu verhindern.

Der alkanhaltige Gasstom 1a z.B. Methanstrom wird mit einer Geschwindigkeit bzw. Flussrate in den Reaktor eingeleitet, so dass es nicht nur zur Verwirbelung des Katalysatorbettes im Reaktor kommt sondern das Methan ebenfalls blasenförmig durch das Wirbelschichtbett durchgeführt wird. Durch die Blasenbildung wird der Wärme- und Massentransport im Reaktorbett verstärkt. Neben der blasenförmigen Verwirbelung ist jedoch auch eine homogene Fahrweise des Reaktors, eine schnellen Verwirbelung bzw. Fluidisierung, eine Fahrweise in einer Steigrohr- Fallrohr-Konfiguration oder eine Fahrweise durch pneumatische Förderung möglich.

Eventuell mitgerissener Katalysator aus dem Katalysatorbett 4 bzw. der dem daraus gebildeten Wirbelschichtbett wird durch ein Zyklon 6 aufgefangen, gesammelt und anschließend in das Wirbelschichtbett zurückgeführt, wohingegen das von Feststoffen befreite Gas den Reaktor am oberen Ende des Reaktors durch die Austrittsleitung 7 verlässt. In Abhängigkeit von dem angewendeten Wirbelschichtprozess, unter welchen der Reaktor betrieben wird, kann das Design des Zyklons 6 sich von dem in der Figur 1 gezeigten unterscheiden.

Eine spezifische Ausführungsform des vorliegenden Reaktors lässt sich wie folgt beschreiben. Der Wirbelschicht-Membran Reaktor wurde gestaltet, um mit einer Katalysatormenge zwischen 30 bis 150 g pro Batch betrieben zu werden. Der innere Durchmesser des aus einer FeCr-Legierung hergestellten Reaktors beträgt 56 mm. Der Reaktor kann bei Temperaturen bis zu 1300°C, betrieben werden. Das Legierungsmaterial ist inert und bildet eine inerte Aluminiumoxidschicht auf der äußeren Oberfläche des Reaktors. Eine Packungsschicht aus Kieselerde, die mit einer Metallmasche von 150 µm separiert ist, wird zur Gasverteilung verwendet, um einen ausreichenden Druckabfall für eine problemlose Fluidisierung bereitzustellen.

Die als Membranen verwendeten Schläuche weisen einen Durchmesser von 6 mm auf und sind aus Hastelloy X hergestellt. Der innere Durchmesser der Membranschläuche beträgt 4 mm und die Membranschläuche sind lediglich im letzten Abschnitt innerhalb der katalytischen Wirbelschicht permeabel. Die Böden der Membranschläuche sind aus gesinterten Partikeln hergestellt und bilden eine poröse Schicht mit einer angepassten Porengröße. Die Porengrösse sollte so ausgewählt sein, um ein ausreichend großen Druckabfall und entsprechend eine gleichmäßige Sauerstoffverteilung entlang des Katalysatorbettes bereitzustellen. Der Sauerstoff tritt am oberen Teil der Membranschläuche ein und wird über die permeablen Abschnitte der Membran in das Katalysatorbett verteilt.

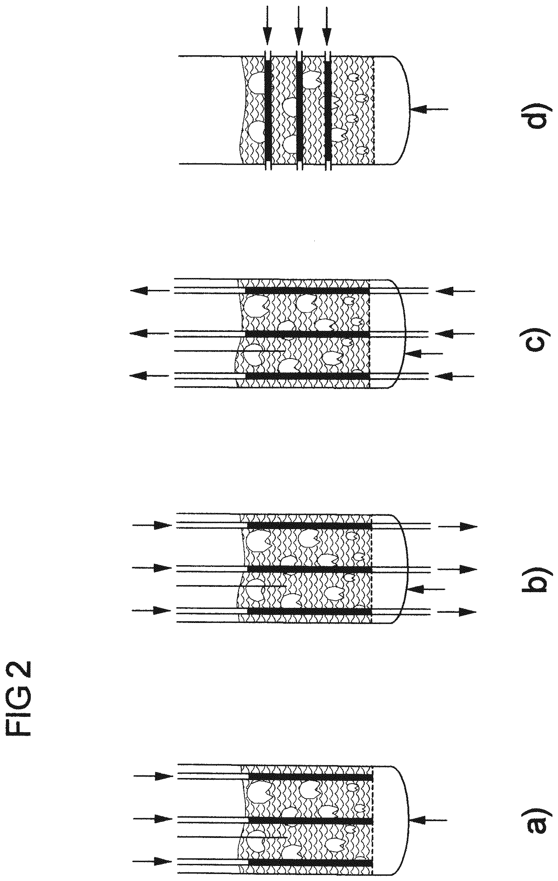

Figuren 2a bis 2d zeigen mögliche Ausführungsformen einer Anordnung der Membranen 5 im Reaktor und deren unterschiedliche Verwendung im vorliegenden Verfahren. In den Ausführungsformen der Figuren 2a bis d sind jeweils drei verschiedene Membranabschnitte parallel zueinander geschaltet bzw. angeordnet.

Die aktiven Abschnitte der Membranen 5 gemäß der Figuren 2a bis c sind dabei jeweils parallel zu der Durchflussrichtung des Alkangasstroms durch den Reaktor und somit vertikal im Reaktor angeordnet. Eine derartige Anordnung ermöglicht den Eintrag des Sauerstoffgasfluss durch die Membranen 5 im Dead-end-Strom (Figur 2a), Gegenstrom (Figur 2b) oder Gleichstrom (Figur 2c) in Bezug auf die Durchflussrichtung des alkanhaltigen Stroms durch den Reaktor.

In einer besonders bevorzugten Ausführungsform ist es auch denkbar, die drei Membranabschnitte vertikal zur Durchflussrichtung des alkanhaltigen Gases und somit horizontal im Reaktor anzuordnen (Figur 2d). Der Eintrag des Sauerstoffstroms erfolgt dann im Kreuzstrom.

Es ist anzumerken, dass das hier gezeigte Wirbelschichtbett nicht nur für die Aufrechterhaltung eines isothermen Betriebes aber auch für die Verstärkung bzw. Verbesserung des Massentransportes von Sauerstoff radial von den Membranen in das Reaktorbett dient, so dass die Selektivität in Bezug auf die gewünschten Produkte weiter erhöht wird. Das vorliegende Verfahren kann in einem weiten Bereich von Verfahrensbedingungen durchgeführt werden, jedoch sind die in der vorliegenden Anmeldung beschriebenen Verfahrensbereiche bevorzugt.

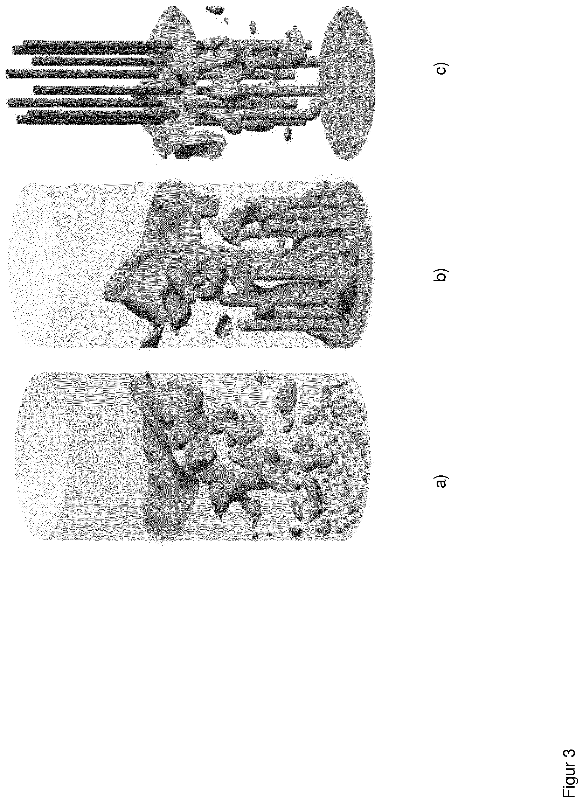

In Figur 3 werden verschiedene Ansätze zum Einführen von Sauerstoff in einen Wirbelschicht-Membran-Reaktor dargestellt und getestet.

So wird in Figur 3a) der Sauerstoff über eine am Reaktorboden angeordneten perforierten Verteiler in das Wirbelbett eingetragen. Der Eintrag des Sauerstoffs erfolgt hier entsprechend nicht über eine Membran, so dass keine feine Sauerstoffverteilung im Reaktor möglich ist.

In Figur 3b) erfolgt der Sauerstoffeintrag über eine am Reaktorboden angeordnete Membran und in Figur 3c) wird Sauerstoff über eine als Membran ausgebildete Eintrittsleitung von oben in den Reaktor eingeführt.

In der folgenden Tabelle 1 sind die Reaktionsausbeuten für alle drei gezeigten Varianten zusammengefasst.

**Tabelle 1: Vergleich der Effizienz der in Figur 3 gezeigten verschiedenen Reaktoranordnungen mit X(CH4)...Methanumsetzung, S(C2).....Selektivität, Y(C2).....Ausbeute**

| **Reaktortyp** | **X(CH4)** | **S(C2)** | **Y (C2)** | **Verbesserung** |
|---|---|---|---|---|
| 3a) Verteiler | 39,2% | 36,4 % | 14,1 % | 12% |
| 3b) Membran am Boden | 36,5 % | 45,1 % | 16,4 % | 30,1 % |
| 3c) Membran als Eintrittsleitung | 31,3 % | 55,4 % | 17,3 % | 37,3 % |

Aus Tabelle 1 ist deutlich zu entnehmen, dass das Einleiten von Sauerstoff in den Wirbelschicht-Membran-Reaktor zu einer massiven Effizienzsteigerung von 12% im Fall 3a) zu 30,1% im Falle von 3b) und 37,3 % im Falle von 3c) im Vergleich zu konventionellen Referenz-Wirbelschicht-Reaktoren führt.

Darüber hinaus zeigt ein Vergleich der Ausführungsformen 3b) und 3c), dass neben der Verwendung einer Membran auch die Anordnung der Membran, z.B. am Reaktorboden oder im Reaktorinneren als Teil der Sauerstoff-Eintrittsleitungen für die Ausbeuteerhöhung von Bedeutung sind. So erhöht sich die Gesamtausbeute bei Anordnung der Membran im Reaktorinneren im Falle 3c) nochmals um ca. 7% im Vergleich zu der Anordnung der Membran im Reaktorboden.

Um die Effizienz und Anwendbarkeit des Wirbelschicht-Membranreaktor-Konzeptes zu verdeutlichen, wird im Folgenden ein Vergleich zwischen der Durchführung eines oxidativen Kopplungsverfahrens in einem Wirbelschichtbett-Reaktor und einem Wirbelschichtbett-Membran-Reaktor beschrieben. Das Zugabeverhältnis und die Temperatur, die in den vorliegenden Beispielen angeführt werden, stellen die bevorzugten Parameter für Wirbelschichtbett-Reaktoren zur oxidativen Methankupplung dar.

### Vergleichsbeispiel

Ein Quarzreaktor mit einem äußeren Durchmesser von 40 mm und einem inneren Durchmesser von 36 mm wird mit einem La₂O₃-CaO-Katalysator mit einer ungefähren Katalysatorpartikelgröße von 150 µm gefüllt. Der Katalysator wurde hergestellt wie von Stansch et al (Industrial Engineering Chemical Research 36, 1979, S. 2568 - 2579) beschrieben. Die statische Höhe des Katalysatorbetts beträgt ca. 5 cm.

Der Reaktor wurde mit einem Gesamtstrom von 2 nl/min einer Gasmischung enthaltend 10 mol% Methan und 4 mol% Sauerstoff gefüllt. Ein derartiger Fluss unter Reaktionsbedingungen ergibt eine lineare Geschwindigkeit, die ca. gleich 2,5 x einer minimalen Fluidisierungsgeschwindigkeit ist.

Der Reaktor wurde auf eine Katalysatorbetttemperatur von ca. 820 °C erwärmt. Das Gas am Austritt wurde mit einem Sick 700 Infrarot-Gasanalysator und einem Perkin-Elmer-Clarus 500 Gaschromatograph analysiert.

Basierend auf näheren Analysen wurde eine Massenbilanz von C₂₊ (Ethan + Ethen) von 14,5 % mit einer Methanumsetzung von 38,8 % und einer Selektivität von 36,5 % erhalten. Der Reaktor konnte isotherm betrieben werden.

### Ausführungsbeispiel

Ein ähnlicher Test mit derselben Katalysator-Temperatur und demselben Dimensionen des Reaktors wurde unter Verwendung eines Wirbelschicht-Membran-Reaktors durchgeführt.

Hierzu wurden 4 keramische Membranen in einer dead-end-Konfiguration mit einem äußeren Durchmesser von 4 mm vertikal in dem Reaktorbehälter befestigt. Der Sauerstoffstrom wurde von oben in den Reaktor eingeleitet. Die durchlässige Schicht bzw. der durchlässige Abschnitt der Membranen wurde in die Wirbelschicht des Katalysators eingetaucht, wobei lediglich 40 mm der Membran gasdurchlässig waren.

Der Strom wurde so in zwei Teile unterteilt, so dass exakt die gleichen Mengen von Methan und Sauerstoff wie in dem oberen Vergleichsbeispiel in die Wirbelschicht eingeführt wurden. Der volumetrische Fluss von 1,33 nl/min enthaltend 15 mol% von Methan wurde von unten eingeführt. Ein volumetrischer Fluss von 0,66 nl/min enthaltend 12,3 mol% Sauerstoff wurde durch die Membranen eingeführt.

Die Durchführung der oxidativen Reaktion in diesem Reaktor führte zu einer Umsetzung von Methan zu 39 %, einer Selektivität von 56 % und einer Ausbeute von 21,8 %. Wie in dem Vergleichsbeispiel konnte der Reaktor ohne Temperaturänderung in dem Katalysatorbett betrieben werden.

Solch eine Verbesserung der Leistung von über 40 % belegt eindeutig das Potential der Verwendung von Wirbelschicht-Membran-Reaktoren zur Verbesserung der Produktausbeute bei oxidativen Kupplungsverfahren.

Des Weiteren benötigen Wirbelschicht-Membran-Reaktoren einen erheblichen Anteil von Katalysatoren und somit einen relativ hohen Durchfluss, so dass das vorliegende Verfahren ohne Probleme an einen industriellen Maßstab angepasst werden kann.

## Patentansprüche

1. Verfahren zur oxidativen Umwandlung von mindestens einem gasförmigen Alkan, bevorzugt von einem C1-C4 Alkan, insbesondere bevorzugt von Methan, in mindestens einem Wirbelschicht-Membran-Reaktor, der mindestens eine erste Eintrittsleitung (1), mehr als eine zweite Eintrittsleitung (3), welche jeweils zumindest abschnittsweise innerhalb des Reaktors in Form einer Membran (5) mit mindestens einer aktiven Membranfläche parallel zueinander angeordnet vorliegen, und mindestens ein Katalysatorbett (4) umfasst,
**gekennzeichnet durch** die Schritte
- Einleiten von mindestens einem ersten Strom (1 a) enthaltend das mindestens eine gasförmige Alkan in das mindestens eine Katalysatorbett (4) im Reaktor über die mindestens eine erste Eintrittsleitung (1), wobei das Einleiten des ersten alkanhaltigen Stroms (1 a) in das mindestens eine Katalysatorbett (4) zur Ausbildung von mindestens einer Katalysator-Wirbelschicht in dem Wirbelschicht-Membran-Reaktor führt, und
- Einleiten von mindestens einem zweiten Strom (2) enthaltend Sauerstoff oder ein sauerstoffhaltiges Gas in den Reaktor über die mehr als eine zweite Eintrittsleitung (3), wobei der zweite Strom (2) über die jeweilige mindestens eine aktive Membranfläche (5) in die mindestens eine Katalysator-Wirbelschicht des Reaktors eingeführt wird.

2. Verfahren nach Anspruch 1, **dadurch gekennzeichnet, dass** die aktive Membranfläche (5) vollständig mit dem Katalysatorbett (4) oder der Katalysator-Wirbelschicht in Berührung kommt.

3. Verfahren nach Anspruch 1 oder 2, **dadurch gekennzeichnet, dass** die Fläche der das Katalysatorbett (4) kontaktierenden aktiven Membranfläche (5) zur Einstellung des Flusses des mindestens zweiten Sauerstoff-enthaltenden Stroms (2) anpassbar ist, insbesondere dass die Fläche der mindestens einen das Katalysatorbett (4) kontaktierenden aktiven Membranfläche (5) mittels einer entlang der Membran beweglichen Hülle einstellbar ist.

4. Verfahren nach einem der vorhergehenden Ansprüche, **dadurch gekennzeichnet, dass** der Stromfluss des mindestens zweiten Sauerstoff enthaltenden Stroms (2) durch die Anordnung und Anzahl der Membranen (5) innerhalb des Reaktors einstellbar ist.

5. Verfahren nach einem der vorhergehenden Ansprüche, **dadurch gekennzeichnet, dass** der Stromfluss des mindestens zweiten Sauerstoff enthaltenden Stroms (2) über die Porengröße der aktiven Membranfläche (5) einstellbar ist, wobei die Porengröße kleiner 500 nm ist und insbesondere 2 bis 20 nm beträgt.

6. Verfahren nach einem der vorhergehenden Ansprüche, **dadurch gekennzeichnet, dass** der mindestens erste alkanhaltige Strom (1a) am Boden des Reaktors in denselbigen eingeleitet wird.

7. Verfahren nach einem der vorhergehenden Ansprüche, **dadurch gekennzeichnet, dass** das Katalysatorbett (4) und damit die daraus gebildete Wirbelschicht aus Katalysatorpartikeln mit einer Größe zwischen 10 µm und 10 mm, bevorzugt zwischen 75 µm und 300 µm besteht.

8. Verfahren nach einem der vorhergehenden Ansprüche, **dadurch gekennzeichnet, dass** die Membran (5) aus einem porösen, perforierten oder anderweitig Gas-durchlässigen Material, insbesondere aus keramischen, metallischen, porösen keramischen, porösen metallischen, perforierten metallischen Material besteht, und/oder mit einem katalytisch aktiven Material beschichtet ist.

9. Verfahren nach einem der vorhergehenden Ansprüche, **dadurch gekennzeichnet, dass** die Membran (5) parallel oder vertikal in Bezug auf die Durchflussrichtung des alkanhaltigen ersten Gasstroms (1a) durch den Reaktor innerhalb des Reaktors angeordnet ist.

10. Verfahren nach einem der vorhergehenden Ansprüche, **dadurch gekennzeichnet, dass** das Verfahren bei Reaktionstemperaturen im Bereich zwischen 350°C und 1300°C, bevorzugt im Bereich zwischen 500°C und 1000°C, insbesondere bevorzugt zwischen 650°C und 900°C durchgeführt wird.

11. Verfahren nach einem der vorhergehenden Ansprüche, **dadurch gekennzeichnet, dass** das Verfahren bei einem Druck im Bereich zwischen 0,2 bar und 100 bar, bevorzugt zwischen 1 bar und 20 bar, insbesondere bevorzugt zwischen 1 bar und 6 bar durchgeführt wird.

12. Verfahren nach einem der vorhergehenden Ansprüche, **dadurch gekennzeichnet, dass** das Verfahren mit weiteren exothermischen oder endothermischen Reaktionen innnerhalb des gleichen Reaktors, in parallelen Reaktoren, oder in einem integrierten Reaktorkonzept kombinierbar ist.

13. Wirbelschicht-Membran-Reaktor zur Durchführung eines Verfahrens nach einem der vorhergehenden Ansprüche umfassend
- mindestens ein Katalysatorbett (4),
- mindestens eine erste Eintrittsleitung (1) zum Einleiten von mindestens einem ersten Strom (1 a) enthaltend mindestens ein erstes Gas, insbesondere ein gasförmiges Alkan, in das mindestens eine in dem Reaktor angeordnete Katalysatorbett (4), und
- mehr als eine zweite Eintrittsleitung (3), welche jeweils zumindest abschnittsweise innerhalb des Reaktors in Form einer Membran (5) mit einer aktiven Membranfläche parallel zueinander angeordnet vorliegen, zum Einleiten von mindestens einem zweiten Strom (2) enthaltend mindestens ein zweites Gas, insbesondere ein sauerstoffhaltiges Gas, über die aktive Membranfläche der zweiten Eintrittsleitung in das mindestens eine Katalysatorbett (4) des Reaktors, wobei
der als Membran (5) ausgebildete Abschnitt der zweiten Eintrittsleitung (3) und damit die aktive Membranfläche mittels einer Steuereinrichtung innerhalb des Reaktors bewegt werden kann..

14. Reaktor nach Anspruch 13, **dadurch gekennzeichnet, dass** die aktive Membranfläche (5) der zweiten Eintrittsleitung (3) vollständig in das Katalysatorbett (4), insbesondere in das verwirbelte Katalysatorbett, eintaucht oder von diesem bedeckt ist.

15. Reaktor nach einem der Ansprüche 13 bis 14, **dadurch gekennzeichnet, dass** der Reaktor mit anderen Reaktortypen, insbesondere Festbettreaktor, simulierten Wanderbettreaktor, oder andere für die oxidative Kopplung geeignet Reaktortypen mit oder ohne Recyclingstrom kombinierbar ist.

## Claims

1. A process for the oxidative conversion of at least one gaseous alkane, preferably of a C1-C4 alkane, in particular preferably of methane, in at least one fluidized-bed membrane reactor which comprises at least one first inlet line (1), more than one second inlet line (3), which at least sectionally each are arranged parallel to each other within the reactor in the form of a membrane (5) with at least one active membrane surface, and at least one catalyst bed (4),
**characterized by** the following steps:
- introducing at least one first stream (1a) containing the at least one gaseous alkane into the at least one catalyst bed (4) in the reactor via the at least one first inlet line (1), wherein the introduction of the first alkane-containing stream (1 a) into the at least one catalyst bed (4) leads to the formation of at least one fluidized catalyst bed in the fluidized-bed membrane reactor, and
- introducing at least one second stream (2) containing oxygen or an oxygen-containing gas into the reactor via the more than one second inlet line (3), wherein the second stream (2) is introduced into the at least one fluidized catalyst bed of the reactor via the respective at least one active membrane surface (5).

2. The process according to claim 1, **characterized in that** the active membrane surface (5) completely gets in contact with the catalyst bed (4) or the fluidized catalyst bed.

3. The process according to claim 1 or 2, **characterized in that** the area of the active membrane surface (5) contacting the catalyst bed (4) is adaptable for adjusting the flow rate of the at least second oxygen-containing stream (2), in particular that the area of the at least one active membrane surface (5) contacting the catalyst bed (4) is adjustable by means of an envelope movable along the membrane.

4. The process according to any of the preceding claims, **characterized in that** the flow rate of the at least second oxygen-containing stream (2) is adjustable by the arrangement and number of the membranes (5) within the reactor.

5. The process according to any of the preceding claims, **characterized in that** the flow rate of the at least second oxygen-containing stream (2) is adjustable via the pore size of the active membrane surface (5), wherein the pore size is smaller than 500 nm and in particular is 2 to 20 mm.

6. The process according to any of the preceding claims, **characterized in that** the at least first alkane-containing stream (1a) is introduced into the reactor at the bottom of the same.

7. The process according to any of the preceding claims, **characterized in that** the catalyst bed (4) and hence the fluidized bed formed therefrom consists of catalyst particles with a size between 10 µm and 10 mm, preferably between 75 µm and 300 µm.

8. The process according to any of the preceding claims, **characterized in that** the membrane (5) consists of a porous, perforated or otherwise gas-permeable material, in particular of ceramic, metallic, porous ceramic, porous metallic, perforated metallic material, and/or is coated with a catalytically active material.

9. The process according to any of the preceding claims, **characterized in that** the membrane (5) is arranged within the reactor in parallel or vertically with respect to the flow direction of the alkane-containing first gas stream (1 a) through the reactor.

10. The process according to any of the preceding claims, **characterized in that** the process is carried out at reaction temperatures in the range between 350 °C and 1300 °C, preferably in the range between 500 °C and 1000 °C, in particular preferably between 650 °C and 900 °C.

11. The process according to any of the preceding claims, **characterized in that** the process is carried out at a pressure in the range between 0.2 bar and 100 bar, preferably between 1 bar and 20 bar, in particular preferably between 1 bar and 6 bar.

12. The process according to any of the preceding claims, **characterized in that** the process can be combined with further exothermal or endothermal reactions within the same reactor, in parallel reactors, or in an integrated reactor concept.

13. A fluidized-bed membrane reactor for carrying out a process according to any of the preceding claims, comprising:
- at least one catalyst bed (4),
- at least one first inlet line (1) for introducing at least one first stream (1a) containing at least one first gas, in particular a gaseous alkane, into the at least one catalyst bed (4) arranged in the reactor, and
- more than one second inlet line (3), which at least sectionally each is arranged parallel to each other within the reactor in the form of a membrane (5) with an active membrane surface, for introducing at least one second stream (2) containing at least one second gas, in particular an oxygen-containing gas, via the active membrane surface of the second inlet line into the at least one catalyst bed (4) of the reactor, wherein
the portion of the second inlet line (3) formed as membrane (5) and hence the active membrane surface can be moved within the reactor by means of a control device.

14. The reactor according to claim 13, **characterized in that** the active membrane surface (5) of the second inlet line (3) fully is immerged into the catalyst bed (4), in particular into the fluidized catalyst bed, or is covered by the same.

15. The reactor according to any of claims 13 to 14, **characterized in that** the reactor can be combined with other types of reactor, in particular fixed-bed reactor, simulated moving-bed reactor, or other types of reactor suitable for oxidative coupling with or without recycling stream.

## Revendications

1. Procédé pour la conversion oxydative d'au moins un alcane gazeux, de préférence d'un C1-C4 alcane, de manière particulièrement préférée du méthane, dans au moins un réacteur à membrane et à couche fluidisée, qui comprend au moins une première conduite d'entrée (1), plus d'une seconde conduite d'entrée (3), qui sont disposées dans chaque cas au moins par sections à l'intérieur du réacteur parallèlement entre elles sous forme d'une membrane (5) avec au moins une surface de membrane active, et au moins un lit de catalyseur (4), **caractérisé par** les étapes
- introduction d'au moins un premier courant (la) contenant le au moins un alcane gazeux dans le au moins un lit de catalyseur (4) dans le réacteur par le biais de la au moins une première conduite d'entrée (1), où l'introduction du premier courant contenant au moins un alcane (la) dans le au moins un lit de catalyseur (4) conduit à la formation d'au moins une couche fluidisée de catalyseur dans le réacteur à membrane et à couche fluidisée, et
- introduction d'au moins un second courant (2) contenant de l'oxygène ou un gaz contenant de l'oxygène dans le réacteur par le biais de la plus d'une seconde conduite d'entrée (3), où le second courant (2) est introduit par le biais de la au moins une surface de membrane active (5) correspondante dans la au moins une couche fluidisée de catalyseur du réacteur.

2. Procédé selon la revendication 1 **caractérisé en ce que** la surface de membrane active (5) vient totalement en contact avec le lit de catalyseur (4) ou la couche fluidisée de catalyseur.

3. Procédé selon la revendication 1 ou 2 **caractérisé en ce que** la surface de la surface de membrane active (5) qui entre en contact avec le lit de catalyseur (4) peut être adaptée pour l'ajustement du flux du au moins second courant contenant de l'oxygène (2), en particulier **en ce que** la surface de la au moins une surface de membrane active (5) qui entre en contact avec le lit de catalyseur (4) peut être ajustée au moyen d'une gaine mobile le long de la membrane.

4. Procédé selon l'une des revendications précédentes **caractérisé en ce que** le flux de courant du au moins second courant contenant de l'oxygène (2) peut être ajusté par la disposition et le nombre des membranes (5) à l'intérieur du réacteur.

5. Procédé selon l'une des revendications précédentes **caractérisé en ce que** le flux de courant du au moins second courant contenant de l'oxygène (2) peut être ajusté par le biais de la dimension de pores de la surface de membrane active (5), où la dimension de pores est inférieure à 500 nm et est égale en particulier à 2 à 20 nm.

6. Procédé selon l'une des revendications précédentes **caractérisé en ce que** le au moins premier courant contenant au moins un alcane (la) est introduit dans le réacteur au fond de celui-ci.

7. Procédé selon l'une des revendications précédentes **caractérisé en ce que** le lit de catalyseur (4) et ainsi la couche fluidisée formée par celui-ci consistent en particules de catalyseur d'une dimension entre 10 µm et 10 mm, de préférence entre 75 µm et 300 µm.

8. Procédé selon l'une des revendications précédentes **caractérisé en ce que** la membrane (5) consiste en un matériau poreux, perforé ou perméable aux gaz d'une autre manière, en particulier en matériau céramique, métallique, céramique poreux, métallique poreux, métallique perforé, et/ou est revêtue d'un matériau actif du point de vue catalytique.

9. Procédé selon l'une des revendications précédentes **caractérisé en ce que** la membrane (5) est disposée à l'intérieur du réacteur parallèlement ou verticalement par rapport à la direction de passage du courant gazeux contenant au moins un alcane (la) à travers le réacteur.

10. Procédé selon l'une des revendications précédentes **caractérisé en ce que** le procédé est mis en oeuvre à des températures de réaction dans le domaine entre 350°C et 1300°C, de préférence dans le domaine entre 500°C et 1000°C, de manière particulièrement préférée entre 650°C et 900°C.

11. Procédé selon l'une des revendications précédentes **caractérisé en ce que** le procédé est mis en oeuvre à une pression dans le domaine entre 0,2 bar et 100 bar, de préférence entre 1 bar et 20 bar, de manière particulièrement préférée entre 1 bar et 6 bar.

12. Procédé selon l'une des revendications précédentes **caractérisé en ce que** le procédé peut être combiné avec d'autres réactions exothermiques ou endothermiques à l'intérieur du même réacteur, dans des réacteurs parallèles ou dans un concept de réacteurs intégrés.

13. Réacteur à membrane et à couche fluidisée pour la mise en oeuvre d'un procédé selon l'une des revendications précédentes comprenant
- au moins un lit de catalyseur (4),
- au moins une première conduite d'entrée (1) pour l'introduction d'au moins un premier courant (la) contenant au moins un premier gaz, en particulier un alcane gazeux, dans le au moins un lit de catalyseur (4) disposé dans le réacteur, et
- plus d'une seconde conduite d'entrée (3), qui sont disposées dans chaque cas au moins par sections à l'intérieur du réacteur parallèlement entre elles sous forme d'une membrane (5) avec une surface de membrane active, pour l'introduction d'au moins un second courant (2) contenant au moins un second gaz, en particulier un gaz contenant de l'oxygène, par le biais de la surface de membrane active de la seconde conduite d'entrée dans le au moins un lit de catalyseur (4) du réacteur, où
la section de la seconde conduite d'entrée (3) agencée sous forme de membrane (5) et ainsi la surface de membrane active peuvent être déplacées au moyen d'un dispositif de commande à l'intérieur du réacteur.

14. Réacteur selon la revendication 13 **caractérisé en ce que** la surface de membrane active (5) de la seconde conduite d'entrée (3) plonge totalement dans le lit de catalyseur (4), en particulier dans le lit de catalyseur fluidisé, ou est totalement recouverte par celui-ci.

15. Réacteur selon l'une des revendications 13 à 14 **caractérisé en ce que** le réacteur peut être combiné avec d'autres types de réacteurs, en particulier un réacteur à lit fixe, un réacteur à lit mobile simulé, ou d'autres types de réacteurs appropriés pour le couplage oxydatif avec ou sans courant de recyclage.
